# EUROPEAN PATENT SPECIFICATION

(11) **EP 1 165 031 B1**
(45) Date of publication and mention of the grant of the patent: **08.06.2005**
(21) Application number: 00906573.1
(22) Date of filing: 14.03.2000
(51) Int. Cl.: A61K 7/48

(54) **RESORCINOL COMPOSITION**
RESORCINENTHALTENDE ZUSAMMENSETZUNG
COMPOSITION DE RESORCINOL

(30) Priority: 22.03.1999 US 125553 P
(43) Date of publication of application: 02.01.2002
(73) Proprietor: PFIZER INC., New York, N.Y. 10017 (US)
(72) Inventor: COLLINGTON, Eric William, Knebworth, Hertfordshire SG3 6AH (GB)
(74) Representative: Wood, David John
(86) International application number: PCT/IB2000/000278
(87) International publication number: WO 2000/056279

(56) References cited:
- EP-A- 0 341 664
- EP-A- 0 904 774
- PATENT ABSTRACTS OF JAPAN vol. 016, no. 470 (C-0990), 30 September 1992 (1992-09-30) & JP 04 169511 A (POLA CHEM IND INC;OTHERS: 01), 17 June 1992 (1992-06-17)
- DATABASE WPI Section Ch, Week 199307 Derwent Publications Ltd., London, GB; Class D21, AN 1993-055163 XP002087694 & JP 05 004905 A (POLA CHEM IND INC), 14 January 1993 (1993-01-14)

## Description

The present invention relates to the use of a resorcinol derivative, *i.e*., 4-(2,4-dihydroxyphenyl)cyclohexanol, as a skin lightening agent.

The terms "lightening agent" and "depigmentation agent" are used interchangeably throughout this document.

Skin color in humans arises from a complex series of cellular processes that are carried out within a unique population of cells called melanocytes. Melanocytes are located in the lower part of the epidermis, and their function is to synthesize a pigment, melanin, which protects the body from the damaging effects of ultraviolet radiation.

When skin is exposed to ultraviolet radiation, such as that contained in sunlight, melanocytes increase their synthesis of melanin. Melanin is deposited in melanosomes, which are vesicles found within the cell. The melanosomes are extruded from the cell and carried to the surface of the skin by keratinocytes, which internalize the melanin containing melanosomes. The end result is that the visible layers of the skin exhibit a brown color typically known as a "tan". The darkness of the color observed in the skin is proportionate to the amount of melanin synthesized by melanocytes and transferred to the keratinocytes.

The mechanism by which skin pigmentation is formed, *i.e*., melanogenesis, is particularly complex and schematically involves the following main steps: Tyrosine→L-Dopa→Dopaquinone→Dopachrome→Melanins. The first two reactions in this series are catalyzed by the enzyme tyrosinase. The activity of tyrosinase is promoted by the action of α-melanocyte stimulating hormone or UV rays to have melanin eventually formed as chromatism in the skin. It is well established that a substance has a depigmenting effect if it acts directly on the vitality of the epidermal melanocytes where melanogenesis normally occurs and/or if it interferes with one of the stages in melanin biosynthesis. The active compounds that are employed in the various methods and compositions of this invention inhibit tyrosinase and thus inhibit or decrease melanin biosynthesis.

There is a strong demand for agents that enable acquired deposition sites, such as spots or freckles, to be restored to a normal skin color. For this purpose, a variety of agents and methods have been developed and put on the market. Examples of such methods are (a) a method wherein vitamin C (L-ascorbic acid) having good reducing ability is administered orally in large amounts, (b) a method wherein glutathione is administered parenterally; (c) a method wherein a peroxide, such as hydrogen peroxide, zinc peroxide, sodium peroxide and the like, which is believed to have the bleaching action of melamine, is administered: and (d) a method wherein vitamin C or cysteine is administered topically in the form of an ointment, cream, lotion or the like. Vitamin C has a problem with respect to stability and becomes so unstable in water-containing systems that they will cause changes in odor and color. Thiol compounds such as glutathione and cysteine do not exhibit a satisfactory depigmental effect since the development of the effect is very slow.

The substances in widest use at the present time as depigmentors are, in particular, hydroquinone and its derivatives, particularly its ethers such as hydroquinone monomethyl ether. These compounds, while effective, are known to produce side effects that can be dangerous. Hydroquinone, use of which is limited to a concentration of 2%, is both irritating and cytotoxic to the melanocyte.

United States Patent 4,526,179 refers to certain hydroquinone fatty esters that have good activity and are less irritating and more stable than hydroquinone.

Japanese Patent Application No. 27909/86 refers to other hydroquinone derivatives that do not have the drawbacks of hydroquinone but that have relatively poor efficacy.

United States Patent 5,449,518 refers to 2,5-dihydoxyphenyl carboxylic acid derivatives as skin depigmentation agents.

European Patent Application EP 341,664A1 refers to certain resorcinol derivatives as tyrosinase inhibitors and skin depigmentation agents.

The use of topical depigmention agents that have good efficacy and are harmless is particularly desirable for treating the following: regional hyperpigmentation caused by melanocytic hyperactivity, such as idiopathic melasma occurring either during pregnancy (mask of pregnancy or chloasma) or secondary to estrogen-progesterone contraception; local hyperpigmentation caused by benign melanocytic hyperactivity and proliferation such as lentigo senilis or liver spots; accidental hyperpigmentation such as postlesional photosensitization and scarring; and certain forms of leukoderma such as vitiligo where, if the injured skin cannot be repigmented, the residual zones of normal skin are depigmented to impart a homogeneous white color to the entire skin.

The compound of formula I, *i.e*., 4-(2,4-dihydroxyphenyl)cyclohexanol, and its pharmaceutically acceptable salts, which are used in the various methods and compositions of this invention, are useful in the treatment of the foregoing dermatological conditions as well as other dermatological conditions, some of which are referred to later in this document, for which the subject being treated desires, for medicinal or cosmetic purposes, to lighten or reduce the pigmentation of the skin affected by the condition.

4-(2,4-dihydroxyphenyl)cyclohexanol, and its pharmaceutically acceptable salts, are also useful for the treatment of inflammatory disorders such as psoriasis and acne, and for the treatment of dandruff.

### Summary of Invention

The invention relates to use of the compound 4-(2,4-dihydroxyphenyl)cyclohexanol, which has the formula:

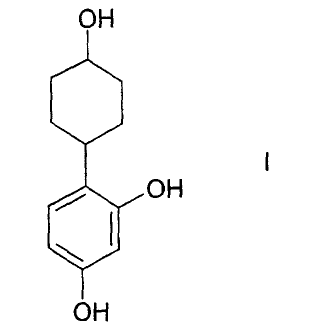

The present invention also relates to use of the pharmaceutically acceptable salts of the compound of formula I.

The present invention also relates to a topical pharmaceutical composition for lightening skin or reducing the pigmentation of skin in a human, comprising an amount of the compound of formula I or a pharmaceutically acceptable salt thereof that is effective in lightening skin or reducing the pigmentation of skin, and a pharmaceutically acceptable carrier.

The present invention also relates to the use of the compound of formula I for the manufacture of a composition for lightening skin or reducing the pigmentation of skin in a human, intended for administering to said human an amount of the compound of formula I or a pharmaceutically acceptable salt thereof that is effective in lightening skin or reducing the pigmentation of skin.

The present invention also relates to a topical pharmaceutical composition for inhibiting tyrosinase in a human, comprising a tyrosinase-inhibiting effective amount of the compound of formula I or a pharmaceutically acceptable salt thereof, and a pharmaceutically acceptable carrier.

The present invention also relates to the use of the compound of formula I for the manufacture of a composition for inhibiting tyrosinase in a human, intended for administering to said mammal a tyrosinase-inhibiting effective amount of the compound of formula I or a pharmaceutically acceptable salt thereof.

The present invention also relates to a topical pharmaceutical composition for lightening skin or reducing the pigmentation of skin in a human, comprising a tyrosinase-inhibiting effective amount of the compound of formula I or a pharmaceutically acceptable salt thereof, and a pharmaceutically acceptable carrier.

The present invention also relates to the use of the compound of formula I for the manufacture of a composition for lightening skin or reducing the pigmentation of skin in a human, intended for administering to said human a tyrosinase-inhibiting effective amount of the compound of formula I or a pharmaceutically acceptable salt thereof.

The present invention also relates to a topical or transdermal pharmaceutical composition for the treatment of an inflammatory disorder such as psoriasis, dermatitis or acne, or dandruff, in a human, comprising an amount of the compound of formula I or a pharmaceutically acceptable salt thereof that is effective in treating such disorder or condition, and a pharmaceutically acceptable carrier.

The present invention also relates to the use of the compound of formula I for the manufacture of a composition for treating inflammatory disorders such as psoriasis, dermatitis or acne, or dandruff, in a human, intended for administering to said human an amount of the compound of formula I or a pharmaceutically acceptable salt thereof that is effective in treating such disorder or condition.

The present invention also relates to a topical or transdermal pharmaceutical composition for the treatment of an inflammatory disorder such as psoriasis, dermatitis or acne in a human, comprising a tyrosinase-inhibiting effective amount of the compound of formula I or a pharmaceutically acceptable salt thereof, and a pharmaceutically acceptable carrier.

The present invention also relates to the use of the compound of formula I for the manufacture of a composition for treating inflammatory disorders, such as psoriasis, dermatitis or acne, in a human, intended for administering to said human a tyrosinase-inhibiting effective amount of the compound of formula I or a pharmaceutically acceptable salt thereof.

The compound of formula I, *i.e*., 4-(2,4-dihydroxyphenyl)cyclohexanol, may exist in different diastereomeric forms. This invention relates to all stereoisomers of 4-(2,4-dihydroxyphenyl)cyclohexanol and mixtures thereof, and to all pharmaceutical compositions and methods of treatment defined above that contain or employ them, respectively.

Formula I includes compounds identical to that depicted above but for the fact that one or more hydrogen, carbon or other atoms are replaced by isotopes thereof. Such compounds may be useful as research and diagnostic tools in metabolism pharmacokinetic studies and in binding assays.

### Detailed Description of the Invention

The compound of formula I may be prepared as described in the following reaction scheme and discussion.

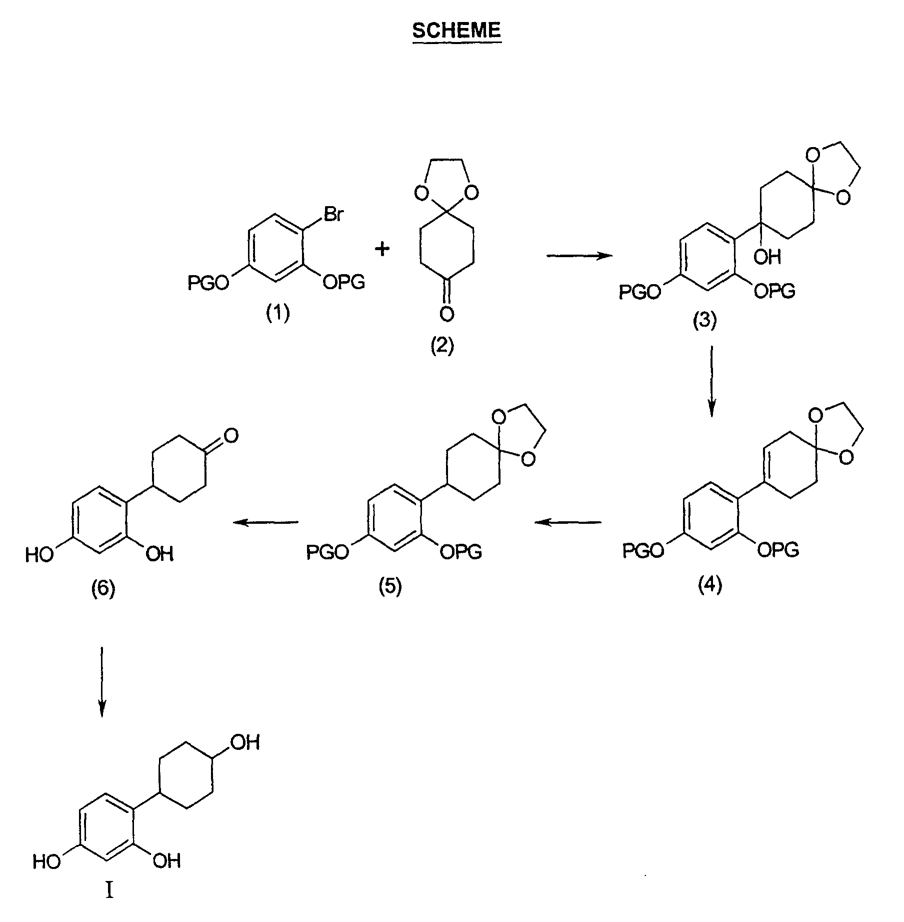

Referring to the scheme, compounds of formula (1) can be formed by protecting commercially available 4-bromoresorcinol. A suitable protecting group such as methoxymethyl (MOM) can be introduced by conventional methods that are well known to those skilled in the art. For example, alkylation of 4-bromoresorcinol can occur with two equivalents of methoxymethyl chloride in the presence of diisopropylamine in a halogenated solvent at about 0°C to room temperature.

The compound of formula (2) is well known and commercially available. The compound of formula (3) can be obtained from the reaction of the compound of formula (1) with n-butyllithium in the presence of N,N,N',N'-tetramethylethylenediamine in an ethereal solvent, followed by the addition of the compound of formula (2). Dehydration of the compound of formula (3) under standard conditions, e.g., heating the compound of formula (3) at about 110°C in a Dean-Stark apparatus in the presence of camphor sulfonic acid in a suitable solvent (e.g., toluene) yields the compound of formula (4). Hydrogenation under standard conditions, e.g., using hydrogen gas and palladium on charcoal in ethanol yields the compound of formula (5). Deprotection under suitable conditions yields the resorcinol of formula (6). The compound of formula (6) can be further derivatised under standard conditions well known to those skilled in the art to yield the resorcinol derivative of formula I. For example, reduction of the compound of formula (6) with a reducing agent such as sodium borohydride in a suitable solvent such as ethanol at a temperature between 0°C and room temperature yields the compound of formula I.

The compound of formula I is weakly acidic in nature and capable of forming base salts with various pharmaceutically acceptable cations. Examples of such salts include the alkali metal and alkaline earth metal salts and, particularly, the sodium and potassium salts. These salts can be prepared by conventional techniques. The chemical bases that are used as reagents to prepare the pharmaceutically acceptable base salts of this invention are those that form non-toxic base salts with the compound of formula I. Such non-toxic base salts include those derived from such pharmaceutically acceptable cations as sodium, potassium, calcium and magnesium, etc. These salts can easily be prepared by treating the corresponding acidic compound with an aqueous solution containing the desired pharmaceutically acceptable cations, and then evaporating the resulting solution to dryness, preferably under reduced pressure. Alternatively, they may also be prepared by mixing lower alkanolic solutions of the acidic compound and the desired alkali metal alkoxide together, and then evaporating the resulting solution to dryness. In either case, stoichiometric quantities of reagents are preferably employed in order to ensure completeness of reaction and maximum yields of the desired final product.

The compound of formula I, *i.e*., 4-(2,4-dihydroxyphenyl)cyclohexanol, and its pharmaceutically acceptable salts (hereinafter referred to as "the active compounds used in this invention") are useful in the treatment of disorders of human pigmentation, including solar and simple lentigines (including age/liver spots), melasma/chloasma and postinflammatory hyperpigmentation. Such compounds reduce skin melanin levels by inhibiting the production of melanin, whether the latter is produced constitutively or in response to UV irradiation (such as sun exposure). Thus, the active compounds used in this invention can be used to reduce skin melanin content in non-pathological states so as to induce a lighter skin tone, as desired by the user. They can also be used in combination with skin peeling agents (including glycolic acid or trichloroacetic acid face peels) to lighten skin tone and prevent repigmentation.

The active compounds used in this invention can also be used in combination with sunscreens (UVA or UVB blockers) to prevent repigmentation, to protect against sun or UV-induced skin darkening or to enhance their ability to reduce skin melanin and their skin bleaching action. Such compounds can also be used in combination with retinoic acid or its derivatives or any compounds that interact with retinoic acid receptors and accelerate or enhance the invention's ability to reduce skin melanin and skin bleaching action.

The active compounds used in this invention can also be used in combination with ascorbic acid, its derivatives, and ascorbic acid-based products (such as magnesium ascorbate) or other products with an anti-oxidant mechanism (such as resveratrol) that accelerate or enhance their ability to reduce skin melanin and their skin bleaching action.

This invention relates both to the use of the compound of formula I for the manufacture of a composition for lightening or reducing the pigmentation of skin in which an active compounds used in this invention and one or more of the other active ingredients referred to above are administered together, as part of the same pharmaceutical composition, as well as the use of the compound of formula I for the manufacture of a composition for administering active ingredients separately as part of an appropriate dose regimen designed to obtain the benefits of the combination therapy. The appropriate dose regimen, the amount of each dose administered, and specific intervals between doses of each active agent will depend upon the specific combination of active agents employed, the condition of the patient being treated, and the nature and severity of the disorder or condition being treated. Such additional active ingredients will generally be administered in amounts less than or equal to those for which they are effective as single topical therapeutic agents. The FDA-approved dosages for such active agents that have received FDA approval for administration to humans are publicly available.

The active compounds used in this invention are generally administered in the form of a pharmaceutical composition comprising the compound together with a pharmaceutically acceptable vehicle or diluent. Such composition is generally formulated in a conventional manner utilizing solid or liquid vehicles or diluents as appropriate for topical administration, in the form of solutions, gels, creams, jellies, pastes, lotions, ointments, salves and the like. Examples of vehicles for application of the active compounds of this invention include an aqueous or water-alcohol solution, an emulsion of the oil-in-water or water-in-oil type, an emulsified gel, or a two-phase system. Preferably, the compositions according to the invention are in the form of lotions, creams, milks, gels, masks, microspheres or nanospheres, or vesicular dispersions. In the case of vesicular dispersions, the lipids of which the vesicles are made can be of the ionic or nonionic type, or a mixture thereof.

As used herein, a "skin-lightening or pigmentation reducing amount of the compound of formula I", and the like, means an amount or concentration of the compound capable of detectably lightening skin or reducing pigmentation in a human, as determined by any standard assay.

As used herein, a "tyrosinase-inhibiting effective amount of the compound of formula I", and the like, means an amount or concentration of the compound capable of detectably inhibiting tyrosinase activity in a human, as determined by any standard assay.

As used herein, an "amount of the compound of formula I capable of treating an inflammatory disorder such as psoriasis, dermatitis or acne, or treating dandruff", and the like, means an amount or concentration of the compound capable of detectably ameliorating, reducing, eliminating, slowing, or preventing the progression of, any symptom or condition associated with or caused by such disorder or condition, in a human, as determined by any standard assay.

In the depigmenting compositions according to the present invention, the concentration of the active compounds used in this invention is generally between 0.01 and 10%, preferably between 0.1 and 10%, relative to the total weight of the composition.

The compositions of this invention can optionally also contain a moistener, a surfactant, keratolytic, an anti-inflammatory agent, a complexing agent, an antioxidant, a preservative, a fragrance, or a sunscreen, or a combination thereof.

The ability of the active compounds used in this invention to inhibit tyrosinase can be determined using any of the following procedures.

### 1. Tyrosinase (DOPA oxidase) assay using cell lysate:

Human melanoma cell line, SKMEL 188 (licensed from Memorial Sloan-Kettering), is used in the cell lysate assay and the screen. In the assay, compounds and L-dihydroxyphenylalanine (L-DOPA) (100 µg/ml) are incubated with the cell lysates containing human tyrosinase for 8 hr before the plates are read at 405 nm. Potency of the compounds in DOPA oxidase assay is correlated very well with that in tyrosine hydroxylase assay using ³H-tyrosine as a substrate. 4-(2,4-dihydroxyphenyl)cyclohexanol, when tested in this assay, exhibited an IC₅₀ of 0.2 µM.

### 2. Melanin assay in human primary melanocytes:

Compounds are incubated with human primary melanocytes in the presence of α-melanocyte stimulating hormone (α-MSH) for 2-3 days. Cells are then lysed with sodium hydroxide and sodium dodecyl sulfate (SDS) and melanin signals are read at 405 nm. Alternatively, ¹⁴C-DOPA is added to the cells in combination with tyrosinase inhibitors and acid-insoluble ¹⁴C-melanin is quantitated by a scintillation counter. IC₅₀ reflects the inhibitory potency of the compound in the new melanin synthesis that was stimulated by α-MSH.

### 3. Tyrosine kinase assay (TK):

TK assays can be performed using purified tyrosine kinases domain of c-met, erb-B2, or IGF-r. A specific antibody against phosphorylated tyrosine residue is used in the assay. Colorimetric signals are generated by horseradish peroxidase, which is conjugated to the antibody.

### 4. Human skin equivalent model:

A mixture of human melanocytes and keratinocytes is grown in an air-liquid interphase. This tissue culture forms a three dimensional structure that histologically and microscopically resembles the human skin epidermis. A test compound is added on top of the cells to mimic topical drug application. After incubation with the compounds (10 µM) for 3 days, the cells are washed extensively and lysed for DOPA oxidase assay.

### 5. IL-1 assay (Interleukin-1 assay):

An IL-1α ELISA assay (R&D system) can be used to evaluate the effect of the compound on IL-1 secretion in a human skin equivalent model. IL-1α is a pro-inflammatory cytokine and plays a role in UV-induced skin inflammation.

### 6. In vivo study:

Black or dark brown guinea pigs with homogeneous skin color can be used in this study. A solution of the test compound (5% in ethanol:propylene glycol, 70:30) and the vehicle control are applied to the animals twice daily, 5 days per week for 4-8 weeks. Using this assay, depigmentation of skin by 4-(2,4-dihydroxyphenyl)cyclohexanol was observed.

The present invention is illustrated by the following example. It will be understood, however, that the invention is not limited to the specific details of this example. Proton nuclear magnetic resonance spectra (¹H NMR) were measured for solutions in d₆-DMSO, CDCl₃ or d₄-MeOH, and peak positions are expressed in parts per million (ppm) downfield from tetramethylsilane (TMS). The peak shapes are denoted as follows: s, singlet; d, doublet; t, triplet; q, quartet, m, multiplet, b, broad.

The following example is illustrative only, and is not intended to limit the scope of the present invention.

### EXAMPLES

### Intermediate 1

### 1-3-Di(methoxymethoxy)-4-bromobenzene

An oven-dried 250 mL round-bottomed flask equipped with magnetic stirrer, under an argon atmosphere, was loaded with 4-bromoresorcinol (9.45 g, 50 mmol) and CH₂Cl₂ (50 ml). The stirred suspension was cooled to 0°C, and diisopropylamine (19.1 ml, 110 mmol) was added in one portion via syringe. Stirring of the red solution was continued for a further 10 min before methyl chloromethyl ether (10.7 ml, 120mmol) was added dropwise *via* syringe ensuring the internal temperature did not exceed 10°C. The resulting yellow solution was then allowed to warm to room temperature overnight. Ammonium hydroxide solution (50mL, 50%) was poured into the reaction vessel and stirring was continued for 1 hr. The mixture was poured into a separating funnel and the phases separated. The aqueous phase was then extracted with CH₂Cl₂ (3x30 ml), and the combined organics washed with brine (20 ml), dried over anhydrous magnesium sulphate, filtered and concentrated *in vacuo* affording an orange oil. Purification was achieved by flash column chromatography, (SiO₂, ethyl acetate/petroleum ether, 1:1, v/v), furnishing the title product (10.7 g, 77%) as a pale yellow oil. δ_{H}(CDCl₃) 7.42(1H, d), 6.88(1H, d), 6.64(1H, dd), 5.24(2H, s), 5.15(2H, s), 3.53(3H, s), 3.48(3H, s).

### Intermediate 2

### 1-Hydroxy-1-(2,4-dihydroxyphenylbis(methoxy methylether))cyclohexan-4-one ethylene ketal

An oven-dried 250 ml round-bottomed flask, equipped with magnetic stirrer, under an argon atmosphere, was loaded with 4-bromoresorcinol-*bis*(methoxymethyl) ether (2.00 g, 7.2 mmol) and THF (50 mL). *N, N, N', N'*- Tetramethylethylene diamine (2.3 ml, 15.2 mmol) was added in one portion via syringe and the stirred solution was cooled to -78°C. n-Butyl lithium (9.5 ml, 15.2 mmol, 1.6M in hexane) was added dropwise via syringe. The resulting yellow solution was stirred for 1 hr at -78°C, and 1,4-cyclohexanedione monoethylene ketal (1.35 g, 8.7 mmol) was added as a solution in THF (25 ml) slowly via syringe. The resulting solution was stirred at -78°C for 1 hr, and then allowed to warm to room temperature overnight. Hydrochloric acid (20 ml, 2M) was added and the reaction mixture stirred vigorously for 15 min. Ethyl acetate (100 ml) was added and the mixture poured into a separating funnel. The phases were separated and the aqueous phase was extracted with ethyl acetate (3x20 ml). The combined organics were washed with brine (20 ml), dried over anhydrous magnesium sulphate, filtered and concentrated affording an orange oil which was purified by flash column chromatography (SiO₂, ethyl acetate/petroleum ether, 45:55, v/v). The title product (1.42 g, 56%) was isolated as a colourless oil. m/z (ES⁺) 337 (M - H₂O + H)⁺; δ_{H} (CDCl₃) 1.61 - 1.64(2H, m), 2.00 - 2.18(6H, m), 3.44(3H, s), 3.48(3H, s), 3.90 - 3.97(4H, m), 5.11(2H, s), 5.24(2H, s), 6.64(1H, dd), 6.82(1H, d), 7.20(1H, d).

### Intermediate 3

### 1-(2,4-Dihydroxyphenylbis(methoxymethylether) cyclohex-1-en-4-one ethylene ketal

1-Hydroxy-1-(2,4-dihydroxyphenyl*bis*(methoxymethylether))cyclohexane-4-one ethylene ketal (1.40 g, 3.95 mmol) was placed in a 50 ml round-bottomed flask equipped with magnetic stirrer and a Dean-Stark apparatus. Toluene (30 ml) was added, followed by camphor sulphonic acid (10 mg). The stirred solution was then heated under reflux for 1 hr, cooled, and saturated aqueous sodium bicarbonate solution (10 ml) was added. The mixture was poured into a separating funnel and the phases separated. The aqueous phase was extracted with ethyl acetate (2x15 ml), and the combined organics were washed with brine (15 ml), dried over anhydrous magnesium sulphate, filtered, and then concentrated *in vacuo* yielding an orange oil that was purified by flash column chromatography (SiO₂, ethyl acetate/petroleum ether, 45:55, v/v) to afford the title product (0.94 g) as a colourless oil. δ_{H} (CDCl₃) 1.84(2H, t), 2.41 - 2.43(2H, m), 2.56 - 2.62(2H, m), 3.47(6H, s), 3.98 - 4.02(4H, m), 5.13(4H, s), 5.58 - 5.63(1H, m), 6.64(1H, dd), 6.78(1 H, d), 7.08(1H, d).

### Intermediate 4

### 1-(2,4-Dihydroxyphenylbis(methoxymethyl ether))cyclohexan-4-one ethylene ketal

1-(2,4-Dihydroxyphenyl*bis*(methoxymethylether)cyclohex-1-en-4-one ethylene ketal (0.950 g, 2.83 mmol) and palladium (200 mg, 10% on carbon) were stirred under an atmosphere of hydrogen for 15 hr. The mixture was then filtered through a plug of Celite, washing with ethyl acetate. The filtrate was evaporated to dryness, affording the title product (0.955 g, 100%) as a colourless oil. δ_{H}(CDCl₃) 1.67 - 1.87 (8H, m), 2.90 - 2.99(1H, m), 3.46(3H, s), 3.48(3H, s), 3.97(4H, s), 5.12(2H, s), 5.18(2H, s), 6.65(1H, dd), 6.78(1H, d), 7.12(1H, d).

### Intermediate 5

### 4-(2, 4-Dihydroxyphenyl)cyclohexanone

A round-bottomed flask equipped with magnetic stirrer was loaded with 1-(2, 4-dihydroxyphenyl*bis*(methoxymethylether))cyclohexan-4-one ethylene ketal (1.30 g, 3.9 mmol) and methanol (15 ml). To the resulting stirred solution was added aqueous HCl (15 ml, 1M) in one portion. After stirring for 1 hr at room temperature, the acid was quenched by adding saturated aqueous sodium bicarbonate solution (10 ml). After stirring vigorously for 10 min, the reaction mixture was transferred to a separating funnel, the phases separated, and the aqueous phase extracted with ethyl acetate (3x20 ml). The combined organic phases were washed with brine and the solvent evaporated. To the slightly wet crude product was added methanol (30 ml) and acidic ion exchange resin (4 g). The resulting mixture was heated under reflux with stirring for 5 hr. Filtering through a plug of Celite, washing with ethyl acetate, followed by removal of solvent *in vacuo*, afforded an orange oil. Purification by flash column chromatography, (SiO₂, ethyl acetate/petroleum ether, 1:1, v/v) furnished the title product as a white powder (0.54 g, 68%). m/z (ES-) 411 (2M - 1); δ_{H}(CD₃OD) 1.94(2H, ddd), 2.16 - 2.23(2H, m), 2.41 (2H, dt), 2.62(1 H, t), 2.63(1 H, t), 6.24(1 H, dd), 6.31 (1 H, d), 6.92(1 H, d).

### (anti)-4-(2,4-Dihydroxyphenyl)cyclohexanol

4-(2, 4-Dihydroxyphenyl)cyclohexanone (18 mg) was placed in a 25 ml round-bottomed flask equipped with magnetic stirrer. Ethanol (5 ml) was added, followed by sodium borohydride (3.3 mg), and the reaction mixture was stirred for 16 hr. Aqueous HCl (20 ml, 1M), followed by ethyl acetate (20 ml), was added, and the organic phase removed and washed with brine (15 ml), dried over anhydrous magnesium sulphate, filtered, and then concentrated *in vacuo*. The residue was purified by flash column chromatography (SiO₂, ethyl acetate/petroleum ether, 60:40, v/v) to afford the title product (14 mg, 78%) as a white solid. m/z (ES⁻) 267 ((M+60) - 1); δ_{H} (CD₃OD) 1.38-1.56 (4H, m), 1.85-1.88 (2H, m), 2.04 - 2.07 (2H, m), 2.80 (1 H, tt), 3.58 - 3.65 (1 H, m), 6.24-6.29 (2H, m), 6.90 (1 H, d).

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims.

## Claims

1. A topical pharmaceutical composition for lightening skin or reducing the pigmentation of skin in a human, comprising an amount of compound of formula I 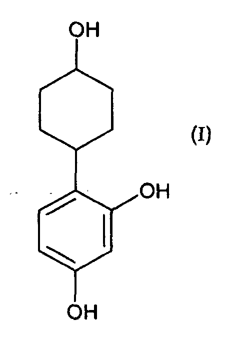 or a pharmaceutically acceptable salt thereof, which amount is effective in lightening skin or reducing the pigmentation of skin, and a pharmaceutically acceptable carrier.

2. The composition of claim 1, further comprising a sunscreen.

3. The composition of claim 1, further comprising resveratrol or another active agent that is an anti-oxidant.

4. The composition of claim 1, further comprising retinoic acid or a derivative of retinoic acid.

5. The composition of claim 1, further comprising glycolic acid, trichloroacetic acid or another skin peeling agent.

6. The composition of claim 1, which is in the form of a water-alcohol solution, an oil-in-water emulsion, a water-in-oil emulsion, an emulsified gel, or a two-phase system.

7. The composition of claim 1, which is in the form of a lotion, cream, milk, gel, jelly, paste, ointment, salve, mask, microspheres, nanospheres, or a vesicular dispersion.

8. The use of a compound of the fomula (I) as defined in claim 1 or of a pharmaceutically acceptable salt thereof for the manufacture of a medicament for lightening skin or reducing the pigmentation of skin in a human.

9. The use of claim 8, in which the compound of formula I or the pharmaceutically acceptable salt thereof is formulated in a water-alcohol solution, an oil-in-water emulsion, a water-in-oil emulsion, an emulsified gel, or a two-phase system.

10. The use of claim 8, in which the compound of formula I or the pharmaceutically acceptable salt thereof is formulated in a lotion, cream, milk, gel, jelly, paste, ointment, salve, mask, microspheres, nanospheres, or a vesicular dispersion.

11. A topical pharmaceutical composition for inhibiting tyrosinase in a human, comprising a tyrosinase-inhibiting effective amount of a compound of formula I 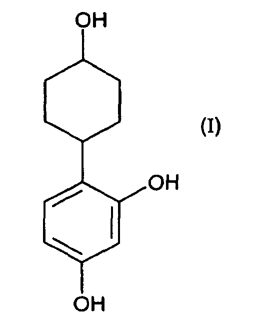 or a pharmaceutically acceptable salt thereof, and a pharmaceutically acceptable carrier.

12. The composition of claim 11, which is in the form of a water-alcohol solution, an oil-in-water emulsion, a water-in-oil emulsion, an emulsified gel, or a two-phase system.

13. The composition of claim 11, which is in the form of a lotion, cream, milk, gel, jelly, paste, ointment, salve, mask, microspheres, nanospheres, or a vesicular dispersion.

14. The use of a compound of the formula (I) as defined in claim 1 or of a pharmaceutically acceptable salt thereof for the manufacture of a medicament for inhibiting tyrosinase in a human.

15. The use of claim 14, in which the compound of formula I or the pharmaceutically acceptable salt thereof is formulated in a water-alcohol solution, an oil-in-water emulsion, a water-in-oil emulsion, an emulsified gel, or a two-phase system.

16. The use of claim 14, in which the compound of formula I or the pharmaceutically acceptable salt thereof is formulated in a lotion, cream, milk, gel, jelly, paste, ointment, salve, mask, microspheres, nanospheres, or a vesicular dispersion.

17. A topical pharmaceutical composition for lightening skin or reducing the pigmentation of skin in a human, comprising a tyrosinase-inhibiting effective amount of a compound of formula I as defined in claim 1, or a pharmaceutically acceptable salt thereof, and a pharmaceutically acceptable carrier.

18. A topical or transdermal pharmaceutical composition for treating an inflammatory disorder or dandruff in a human, comprising an amount of compound of formula I as defined in claim 1 or a pharmaceutically acceptable salt thereof, which amount is effective in treating such disorder, and a pharmaceutically acceptable carrier.

19. The composition of claim 18 wherein the inflammatory disorder is psoriasis, dermatitis or acne.

20. The use of a compound of the formula (I) as defined in claim 1, or of a pharmaceutically acceptable salt thereof, for the manufacture of a medicament for treating an inflammatory disorder or dandruff in a human.

21. The use of claim 20, wherein the inflammatory disorder is psoriasis, dermatitis or acne.

22. A topical or transdermal pharmaceutical composition for treating an inflammatory disorder in a human, comprising a tyrosinase-inhibiting effective amount of a compound of formula I as defined in claim 1, or a pharmaceutically acceptable salt thereof, and a pharmaceutically acceptable carrier.

## Patentansprüche

1. Topische pharmazeutische Zusammensetzung zur Hautaufhellung oder Minderung der Pigmentierung der Haut beim Menschen, umfassend eine Verbindung der Formel I 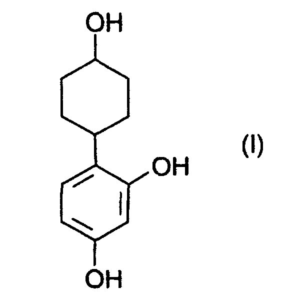 oder deren pharmazeutisch akzeptables Salz in einer zur Hautaufhellung oder Minderung der Pigmentierung der Haut wirksamen Menge sowie einen pharmazeutisch akzeptablen Träger.

2. Zusammensetzung nach Anspruch 1, weiter umfassend ein Sonnenschutzmittel.

3. Zusammensetzung nach Anspruch 1, weiter umfassend Resveratrol oder einen anderen, ein Antioxidans darstellenden Wirkstoff.

4. Zusammensetzung nach Anspruch 1, weiter umfassend Retinolsäure oder deren Derivat.

5. Zusammensetzung nach Anspruch 1, weiter umfassend Glykolsäure, Trichloressigsäure oder einen anderen Hautpeeling-Wirkstoff.

6. Zusammensetzung nach Anspruch 1 in Form einer Wasser-Alkohol-Lösung, einer Öl-in-Wasser-Emulsion, einer Wasser-in-Öl-Emulsion, eines emulgierten Gels oder eines zweiphasigen Systems.

7. Zusammensetzung nach Anspruch 1 in Form einer Lotion, einer Creme, einer Milch, eines Gels, eines Gelees, einer Paste, einer Salbe, Wundsalbe, einer Maske, eines "Puders" aus Mikropartikeln (microspheres), eines "Puders" aus Nanopartikeln (nanospheres) oder einer Vesikeldispersion (vesicular dispersion).

8. Verwendung einer Verbindung der Formel I gemäß Anspruch 1 oder deren pharmazeutisch akzeptablen Salzes zur Herstellung eines Arzneimittels zur Hautaufhellung oder zur Minderung der Pigmentierung der Haut beim Menschen.

9. Verwendung nach Anspruch 8, wobei die Verbindung der Formel I oder deren pharmazeutisch akzeptables Salz in einer Wasser-Alkohol-Lösung, einer Öl-in-Wasser-Emulsion, einer Wasser-in-Öl-Emulsion, einem emulgierten Gel oder einem zweiphasigen System formuliert ist.

10. Verwendung nach Anspruch 8, wobei die Verbindung der Formel I oder deren pharmazeutisch akzeptables Salz in einer Lotion, einer Creme, einer Milch, eines Gels, eines Gelees, einer Paste, einer Salbe, Wundsalbe, einer Maske, eines "Puders" aus Mikropartikeln (microspheres), eines "Puders" aus Nanopartikeln (nanospheres) oder einer Vesikeldispersion (vesicular dispersion) formuliert ist.

11. Topische pharmazeutische Zusammensetzung zur Hemmung der Tyrosinase beim Menschen, umfassend eine zur Hemmung der Tyrosinase wirksame Menge der Verbindung der Formel I 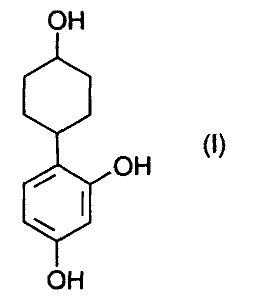 oder deren pharmazeutisch akzeptablen Salzes und einen pharmazeutisch akzeptablen Träger.

12. Zusammensetzung nach Anspruch 11 in Form einer Wasser-Alkohol-Lösung, einer Öl-in-Wasser-Emulsion, einer Wasser-in-Öl-Emulsion, eines emulgierten Gels oder eines zweiphasigen Systems.

13. Zusammensetzung nach Anspruch 11 in Form einer Lotion, einer Creme, einer Milch, eines Gels, eines Gelees, einer Paste, einer Salbe, Wundsalbe, einer Maske, eines "Puders" aus Mikropartikeln (microspheres), eines "Puders" aus Nanopartikeln (nanospheres) oder einer Vesikeldispersion (vesicular dispersion).

14. Verwendung einer Verbindung der Formel I nach Anspruch 1 oder deren pharmazeutisch akzeptablen Salzes zur Herstellung eines Arzneimittels zur Hemmung der Tyrosinase beim Menschen.

15. Verwendung nach Anspruch 14, wobei die Verbindung der Formel I oder deren pharmazeutisch akzeptables Salz in einer Wasser-Alkohol-Lösung, einer Öl-in-Wasser-Emulsion, einer Wasser-in-Öl-Emulsion, einem emulgierten Gel oder einem zweiphasigen System formuliert ist.

16. Verwendung nach Anspruch 14, wobei die Verbindung der Formel I oder deren pharmazeutisch akzeptables Salz in einer einer Lotion, einer Creme, einer Milch, eines Gels, eines Gelees, einer Paste, einer Salbe, Wundsalbe, einer Maske, eines "Puders" aus Mikropartikeln (microspheres), eines "Puders" aus Nanopartikeln (nanospheres) oder einer Vesikeldispersion (vesicular dispersion) formuliert ist.

17. Topische pharmazeutische Zusammensetzung zur Hautaufhellung oder Minderung der Pigmentierung der Haut beim Menschen, umfassend eine zur Hemmung der Tyrosinase wirksame Menge einer Verbindung der Formel I gemäß Anspruch 1 oder deren pharmazeutisch akzeptablen Salzes sowie einen pharmazeutisch akzeptablen Träger.

18. Topische oder transdermale pharmazeutische Zusammensetzung zur Behandlung einer entzündlichen Störung oder zur Behandlung von Schuppen beim Menschen, umfassend eine zur Behandlung einer solchen Störung wirksame Menge der Verbindung der Formel I gemäß Anspruch 1 oder deren pharmazeutisch akzeptablen Salzes sowie einen pharmazeutisch akzeptablen Träger.

19. Zusammensetzung nach Anspruch 18, wobei es sich bei der entzündlichen Störung um Psoriasis, Dermatitis oder Akne handelt.

20. Verwendung einer Verbindung der Formel I gemäß Anspruch 1 oder deren pharmazeutisch akzeptablen Salzes zur Herstellung eines Arzneimittels zur Behandlung einer entzündlichen Störung oder zur Behandlung von Schuppen beim Menschen.

21. Verwendung nach Anspruch 20, wobei es sich bei der entzündlichen Störung um Psoriasis, Dermatitis oder Akne handelt.

22. Topische oder transdermale pharmazeutische Zusammensetzung zur Behandlung einer entzündlichen Störung beim Menschen, umfassend eine zur Hemmung der Tyrosinase wirksame Menge einer Verbindung der Formel I gemäß Anspruch 1 oder deren pharmazeutisch akzeptablen Salzes sowie einen pharmazeutisch akzeptablen Träger.

## Revendications

1. Composition pharmaceutique topique pour l'éclaircissement de la peau ou la réduction de la pigmentation de la peau chez un être humain, comprenant une quantité d'un composé de formule I : 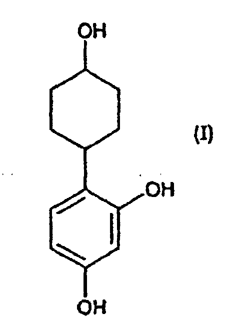 ou d'un de ses sels pharmaceutiquement acceptables, quantité qui est efficace pour l'éclaircissement de la peau ou la réduction de la pigmentation de la peau, et un support pharmaceutiquement acceptable.

2. Composition suivant la revendication 1, comprenant en outre un écran solaire.

3. Composition suivant la revendication 1, comprenant en outre du resvératrol, ou un autre agent actif qui est un antioxydant.

4. Composition suivant la revendication 1, comprenant en outre de l'acide rétinoïque ou un dérivé d'acide rétinoïque.

5. Composition suivant la revendication 1, comprenant en outre de l'acide glycolique, de l'acide trichloracétique ou un autre agent de desquamation cutanée.

6. Composition suivant la revendication 1, qui est sous forme d'une solution aqueuse-alcoolique, d'une émulsion huile-dans-eau, d'une émulsion eau-dans-huile, d'un gel émulsionné ou d'un système biphasique.

7. Composition suivant la revendication 1, qui est sous forme d'une lotion, d'une crème, d'un lait, d'un gel, d'une gelée, d'une pâte, d'une pommade, d'un baume, d'un masque, de microsphères, de nanosphères ou d'une dispersion vésiculaire.

8. Utilisation d'un composé de formule I tel que défini dans la revendication 1 ou d'un de ses sels pharmaceutiquement acceptables pour la production d'un médicament destiné à l'éclaircissement de la peau ou la réduction de la pigmentation de la peau chez un être humain.

9. Utilisation suivant la revendication 8, dans laquelle le composé de formule I ou son sel pharmaceutiquement acceptable est formulé dans une solution aqueuse-alcoolique, une émulsion huile-dans-eau, une émulsion eau-dans-huile, un gel émulsionné ou un système biphasique.

10. Utilisation suivant la revendication 8, dans laquelle le composé de formule I ou son sel pharmaceutiquement acceptable est formulé dans une lotion, une crème, un lait, un gel, une gelée, une pâte, une pommade, un baume, un masque, des microsphères, des nanosphères ou une dispersion vésiculaire.

11. Composition pharmaceutique topique pour inhiber la tyrosinase chez un être humain, comprenant une quantité, efficace pour inhiber la tyrosinase, d'un composé de formule I 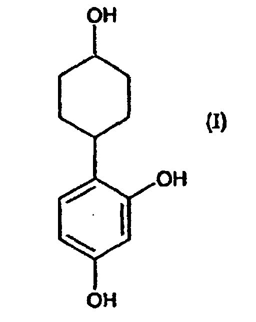 ou d'un de ses sels pharmaceutiquement acceptables, et un support pharmaceutiquement acceptable.

12. Composition suivant la revendication 11, qui est sous forme d'une solution aqueuse-alcoolique, d'une émulsion huile-dans-eau, d'une émulsion eau-dans-huile, d'un gel émulsionné ou d'un système biphasique.

13. Composition suivant la revendication 11, qui est sous forme d'une lotion, d'une crème, d'un lait, d'un gel, d'une gelée, d'une pâte, d'une pommade, d'un baume, d'un masque, de microsphères, de nanosphères ou d'une dispersion vésiculaire.

14. Utilisation d'un composé de formule I telle que définie dans la revendication 1 ou d'un de ses sels pharmaceutiquement acceptables pour la production d'un médicament destiné à inhiber la tyrosinase chez un être humain.

15. Utilisation suivant la revendication 14, dans laquelle le composé de formule I ou son sel pharmaceutiquement acceptable est formulé dans une solution aqueuse-alcoolique, une émulsion huile-dans-eau, une émulsion eau-dans-huile, un gel émulsionné ou un système biphasique.

16. Utilisation suivant la revendication 14, dans laquelle le composé de formule I ou son sel pharmaceutiquement acceptable est formulé dans une lotion, une crème, un lait, un gel, une gelée, une pâte, une pommade, un baume, un masque, des microsphères, des nanosphères ou une dispersion vésiculaire.

17. Composition pharmaceutique topique pour l'éclaircissement de la peau ou pour la réduction de la pigmentation de la peau chez un être humain, comprenant une quantité, efficace pour inhiber la tyrosinase, d'un composé de formule I telle que définie dans la revendication 1, ou d'un de ses sels pharmaceutiquement acceptables, et un support pharmaceutiquement acceptable.

18. Composition pharmaceutique topique ou transdermique pour le traitement d'un trouble inflammatoire ou des pellicules chez un être humain, comprenant une quantité d'un composé de formule I telle que définie dans la revendication 1 ou d'un de ses sels pharmaceutiquement acceptables, quantité qui est efficace dans le traitement de ce trouble, et un support pharmaceutiquement acceptable.

19. Composition suivant la revendication 18, dans laquelle le trouble inflammatoire est le psoriasis, la dermatite ou l'acné.

20. Utilisation d'un composé de formule I telle que définie dans la revendication 1, ou d'un de ses sels pharmaceutiquement acceptables, pour la production d'un médicament destiné au traitement d'un trouble inflammatoire ou des pellicules chez un être humain.

21. Utilisation suivant la revendication 20, dans laquelle le trouble inflammatoire est le psoriasis, la dermatite ou l'acné.

22. Composition pharmaceutique topique ou transdermique pour le traitement d'un trouble inflammatoire chez un être humain, comprenant une quantité, efficace pour inhiber la tyrosinase, d'un composé de formule I telle que définie dans la revendication 1, ou d'un de ses sels pharmaceutiquement acceptables, et un support pharmaceutiquement acceptable.
